# EUROPÄISCHE PATENTANMELDUNG

(11) **EP 4 202 703 A1**
(43) Veröffentlichungstag der Anmeldung: **28.06.2023**
(21) Anmeldenummer: 22214941.1
(22) Anmeldetag: 20.12.2022
(51) Int. Cl.: G06F 12/14, G01N 1/22, G01N 33/00, G06F 21/57

(54) **MESSGERÄT UND VERFAHREN ZUM BETREIBEN EINES MESSGERÄTS**

(30) Priorität: 21.12.2021 AT 510252021
(71) Anmelder: AVL DiTest GmbH, 8020 Graz (AT)
(72) Erfinder: Braunecker, Matthias, 8010 Graz (AT); Leitgeb, Christoph, 8330 Feldbach (AT)
(74) Vertreter: Patentanwälte Pinter & Weiss OG

(57) **Zusammenfassung**

Um die Benutzerfreundlichkeit eines Messgeräts (1) zu verbessern und gleichzeitig eine zuverlässige Erkennung einer Manipulation am Messgerät (1) zu ermöglichen, ist vorgesehen, in der Steuerungseinheit (2) des Messgeräts (1) ein zusätzlicher Sicherheitsprozessor (11) vorgesehen ist, der über eine erste Datenverbindung (D1) mit einem Anwendungsprozessor (3) des Messgeräts (1) verbunden ist und über eine zweite Datenverbindung (D2) mit einem Sicherheitsspeicher (12) verbunden ist und dass der Sicherheitsprozessor (11) dazu ausgebildet ist, einen Logbuch-Eintrag im Sicherheitsspeicher (12) zu speichern, wenn eine, die Erfassung der Messgröße betreffende, Änderung an der Steuerungseinheit (2) vorgenommen wird.

## Beschreibung

Die Erfindung betrifft ein Messgerät zur Erfassung zumindest einer Messgröße, wobei das Messgerät eine Steuerungseinheit mit einem Anwendungsprozessor zur Steuerung des Messgeräts aufweist. Die Erfindung betrifft auch ein Verfahren zum Betreiben eines, zur Erfassung zumindest einer Messgröße ausgebildeten, Messgerätes, wobei das Messgerät durch eine, einen Anwendungsprozessor aufweisende, Steuerungseinheit gesteuert wird.

Messgeräte werden in vielen Ländern für offizielle gesetzliche Messungen verwendet, beispielsweise von Behörden bei der Zulassung von Fahrzeugen oder von Werkstätten im Rahmen der periodischen technischen Fahrzeugüberprüfung. Dafür müssen die Messgeräte in der Regel bei den entsprechenden nationalen Behörden nach nationalen oder internationalen Richtlinien zertifiziert werden (vergleichbar mit Waagen, Tankanlagen, Alkomaten, usw.). Ein Messgerät weist üblicherweise einen oder mehrere Sensoren zur Erfassung von bestimmten Messgrößen auf. Ein solches Messgerät kann beispielsweise ein Abgasmessgerät zur Erfassung einer oder mehrerer Messgrößen im Abgas eines Fahrzeugs sein. Die Messgrößen können beispielsweise bestimmte Schadstoffe wie Kohlendioxid (CO₂), Kohlemonoxid (CO), Stickoxide (Stickstoffmonoxid (NO), Stickstoffdioxid (NO₂)), Partikel (PM), unverbrannte Kohlenwasserstoffe (HC), usw. sein.

Ein Messgerät im Rahmen der Erfindung kann aber beispielsweise auch ein sogenanntes Hochvolt-Messgerät zur Erfassung einer elektrischen Messgröße an einem Fahrzeug sein, was insbesondere für Hybrid- und Elektrofahrzeuge relevant ist, die mit Spannungen im Bereich bis zu mehreren hundert Volt arbeiten. Unter einem Messgerät im Rahmen der Erfindung sind aber natürlich auch noch weitere Messgeräte zu verstehen, insbesondere Messgeräte zur Erfassung einer Messgröße an einem Fahrzeug in einer Werkstätte.

In der Regel weist das Messgerät eine Firmware zur Gerätesteuerung, zur Verarbeitung der erfassten Messwerte der Sensoren und zur Weiterleitung der erfassten Messwerte zu einer Anzeige (z.B. ein Display am Messgerät oder ein Computer mit entsprechender Software) auf. Um eine unerwünschte Änderung der Firmware zu verhindern, muss dabei sichergestellt sein, dass die Firmware nicht einfach manipuliert werden kann, dass also die Firmware beispielsweise nicht einfach durch eine beliebige andere Firmware ersetzt werden kann. Das ist notwendig, um zu verhindern, dass Messungen unter Umständen durch eine geänderte Firmware bewusst gefälscht werden können. Ein völliges Kapseln von Messgeräten ist allerdings ebenso unerwünscht, denn über die Gerätelaufzeit kann es immer wieder zu Änderungen, Erweiterungen oder Korrekturen der Firmware kommen, sodass sichergestellt werden muss, dass es zumindest für den Hersteller eine Möglichkeit gibt, die Firmware zu aktualisieren. Auch eine Kalibrierung der Messgeräte ist von Zeit zu Zeit erforderlich, um dauerhaft eine erforderliche Messgenauigkeit zu erreichen.

Bisher wurde das Problem so gelöst, dass für die Änderung der Firmware ein sogenannter "Jumper" oder Schalter innerhalb eines Gehäuses des Messgeräts gesetzt bzw. betätigt werden musste. Um ins Innere des Gehäuses zu gelangen musste dazu zuerst ein mechanisches Siegel aufgebrochen werden, beispielsweise ein von einem Eichbeamten der Behöre aufgebrachtes Eichsiegel oder eine Plombe. Durch das Zerstören des Siegels konnte eine Öffnung des Gehäuses erkannt werden. Auch der Schalter oder Jumper konnte mit einer Plombe versehen sein, sodass eine Betätigung des Schalters bzw. Jumpers nur nach der Zerstörung der Plombe möglich war. Nach dem Aktualisieren der Firmware musste das Gehäuse und ggf. der Schalter/Jumper natürlich wieder entsprechend versiegelt bzw. plombiert werden, was insbesondere bei behördlichen zugelassenen Messgeräten aufwändig ist, weil dies wiederum z.B. durch einen Eichbeamten durchgeführt werden muss. Abgesehen davon, dass dieser Vorgang unpraktisch in der Handhabung ist, kann es dabei unter Umständen auch zu einer Beeinträchtigung des EMI-/ESD-Schutzes (EMI = electromagnetic interference; ESD= electrostatic discharge) oder zu elektrischen oder mechanischen Beschädigungen des Messgeräts kommen, was natürlich unerwünscht ist, weil dies zu erhöhten Reparatur-, Wartungs- und Instandhaltungskosten führt.

Es ist daher die Aufgabe der Erfindung, die Benutzerfreundlichkeit eines Messgeräts zu verbessern und gleichzeitig eine zuverlässige Erkennung einer Manipulation zu ermöglichen.

Die Aufgabe wird erfindungsgemäß mit dem eingangs genannten Messgerät dadurch gelöst, dass in der Steuerungseinheit ein zusätzlicher Sicherheitsprozessor vorgesehen ist, der über eine erste Datenverbindung mit dem Anwendungsprozessor verbunden ist und über eine zweite Datenverbindung mit einem Sicherheitsspeicher verbunden ist und dass der Sicherheitsprozessor dazu ausgebildet ist, einen Logbuch-Eintrag im Sicherheitsspeicher zu speichern, wenn eine, die Erfassung der Messgröße betreffende, Änderung an der Steuerungseinheit vorgenommen wird. Die Aufgabe wird zudem mit dem eingangs genannten Verfahren dadurch gelöst, dass mit einem in der Steuerungseinheit zusätzlich vorgesehenen und mit dem Anwendungsprozessor kommunizierenden Sicherheitsprozessor einen Logbuch-Eintrag in einem mit dem Sicherheitsprozessor kommunizierenden Sicherheitsspeicher gespeichert wird, wenn eine, die Erfassung der Messgröße betreffende, Änderung an der Steuerungseinheit durchgeführt wird, wobei der Logbuch-Eintrag vorzugsweise einen durch eine Echtzeituhr ermittelten Zeitstempel und/oder die Art der durchgeführten Änderung und/oder einen Benutzernamen des Benutzers, der die Änderung durchgeführt hat, und/oder einen Status der Steuerungseinheit vor der Änderung enthält. An dieser Stelle sei angemerkt, dass es sich bei dem Verfahren um ein teilweise computerimplementiertes Verfahren handelt. Teilweise bedeutet, dass der Schritt der Manipulation beispielsweise durch einen menschlichen Eingriff erfolgen kann, während die übrigen Verfahrensschritte computergestützt ablaufen.

Dadurch wird bei jeder Veränderung an der Steuerungseinheit automatisch ein Logbuch-Eintrag erstellt, beispielsweise eine Protokolldatei, wodurch es zu jeder Zeit nachvollziehbar ist, ob in der Vergangenheit Veränderungen durchgeführt wurden. Dadurch kann beispielsweise die Gefahr verringert werden, dass ggf. behördlich nicht zugelassene Änderungen am Messgerät durchgeführt werden, die unter Umständen zu einer falschen Erfassung der Messgröße oder zu einer Verfälschung der gespeicherten Messdaten führen, weil solche Änderungen zu jeder Zeit nachvollziehbar sind.

In eine vorteilhaften Ausführungsform ist das Messgerät zur Erfassung einer Messgröße an einem Fahrzeug ausgebildet, insbesondere als Abgasmessgerät zur Erfassung einer Messgröße in einem Abgasstrom eines Fahrzeugs. Dadurch kann die Erfindung bei behördlich zugelassenen Messgeräten im Rahmen der periodischen Fahrzeugüberprüfung verwendet werden.

In der Steuerungseinheit ist vorzugsweise eine Echtzeituhr vorgesehen und der Sicherheitsprozessor ist dazu ausgebildet, den Logbuch-Eintrag mit einem durch die Echtzeituhr ermittelten Zeitstempel zu versehen, wobei der Zeitstempel vorzugsweise Datum und Uhrzeit enthält. Dadurch kann jedem Logbuch-Eintrag immer die aktuelle Uhrzeit und das aktuelle Datum hinzugefügt werde, was die Nachvollziehbarkeit von Änderungen bzw. Manipulationen erleichtert.

Der Anwendungsprozessor kann beispielsweise eine Anwendungsfirmware enthalten und die, die Erfassung der Messgröße betreffende, Änderung an der Steuerungseinheit kann in diesem Fall eine Änderung der Anwendungsfirmware umfassen. Dadurch können ggf. unerlaubte Änderungen an der Firmware nachvollzogen werden.

Der Anwendungsprozessor enthält vorzugsweise einen Bootloader, der dazu ausgebildet ist, die Änderung der Anwendungsfirmware durchzuführen, wobei der Bootloader einen unveränderbaren Bootloader-Code aufweist. Dadurch wird gewährleistet, dass keine Änderungen am Bootloader durchgeführt werden können, die ggf. Auswirkungen auf die Erfassung der Messgröße haben.

Vorzugsweise ist der Bootloader dazu ausgebildet, bei einer Änderung der Anwendungsfirmware einen Prüfwert für die geänderte Anwendungsfirmware zu erzeugen und an den Sicherheitsprozessor zu übermitteln und der Sicherheitsprozessor ist dazu ausgebildet, den erhaltenen Prüfiniert im Sicherheitsspeicher zu speichern. Dabei kann es weiters vorteilhaft sein, wenn der Bootloader dazu ausgebildet ist, bei der Inbetriebnahme des Messgeräts vor dem Start der Anwendungsfirmware einen Prüfwert für die aktuelle Anwendungsfirmware zu ermitteln und mit dem zuletzt im Sicherheitsspeicher gespeicherten Prüfwert zu vergleichen, um ein Vergleichsergebnis zu ermitteln. Dadurch kann in einfacher Weise geprüft werden, ob Änderungen an der Anwendungsfirmware durchgeführt wurden.

Es ist weiters vorteilhaft, wenn der Sicherheitsprozessor eine unveränderbare Sicherheitssoftware oder unveränderbare Sicherheitsfirmware aufweist. Dadurch können ggf. unbefugte Änderungen am Sicherheitsprozessor verhindert werden, die unter Umständen die Speicherung der Logbuch-Einträge beeinflussen.

In der Steuerungseinheit ist vorzugsweise auch ein Anwendungsspeicher zum Speichern von Anwendungsdaten vorgesehen, der über eine dritte Datenverbindung mit dem Anwendungsprozessor verbunden ist und dass die, die Erfassung der Messgröße betreffende, Änderung an der Steuerungseinheit eine Änderung der im Anwendungsspeicher gespeicherten Anwendungsdaten umfasst. Die Anwendungsdaten können beispielsweise Messdaten der erfassten Messgröße und/oder Kalibrierparameter des Messgeräts und/oder Metadaten über die erfassten Messdaten enthalten. Dadurch können auch Änderungen an den bereits gespeicherten Messdaten, Kalibrierdaten oder an den Metadaten nachvollzogen werden. Damit können ggf. unerlaubte Manipulationen an den Messdaten festgestellt werden oder eine Änderung eines für die Erfassung der Messdaten verantwortlichen Benutzers, usw.

Der Anwendungsprozessor ist vorzugsweise dazu ausgebildet, Anwendungsdaten an den Sicherheitsprozessor zu übermitteln, wobei der Sicherheitsprozessor dazu ausgebildet ist, die erhaltenen Anwendungsdaten im Sicherheitsspeicher zu speichern und/oder im Sicherheitsspeicher gespeicherte Anwendungsdaten an den Anwendungsprozessor zu übermitteln. Dadurch kann beispielsweise bei einer Veränderung der Anwendungsdaten in einfacher Weise die zuletzt gespeicherten Anwendungsdaten wiederhergestellt werden.

Der Logbuch-Eintrag enthält vorzugsweise die Art der durchgeführten Änderung an der Steuerungseinheit und/oder einen Benutzernamen des Benutzers, der die Änderung an der Steuerungseinheit durchgeführt hat, und/oder einen Status der Steuerungseinheit vor der Änderung an der Steuerungseinheit. Dadurch wird die Nachvollziehbarkeit der durchgeführten Änderungen erleichtert, sodass eine Fehlererkennung und- Fehlerbehebung einfacher erfolgen kann.

Das Messgerät weist vorzugsweise auch eine Anzeigeeinrichtung auf, die dazu ausgebildet ist, gespeicherte Logbuch-Einträge und/oder das ermittelte Vergleichsergebnis der Prüfwerte und/oder Anwendungsdaten anzuzeigen, wobei die Anzeigeeinrichtung vorzugsweise zumindest eine Signallampe oder einen Bildschirm aufweist. Dadurch kann ein Benutzer in einfacher Weise überprüfen, ob eine ggf. unerwünschte Manipulation am Messgerät durchgeführt wurde.

Das Messgerät weist in vorteilhafter Weise auch eine Benutzerschnittstelle auf, über die Funktionen des Messgeräts von einem Benutzer steuerbar sind, wobei die Benutzerschnittstelle vorzugsweise einen Touchscreen oder eine Tastatur aufweist. Dadurch wird die Bedienbarkeit für Benutzer verbessert.

Das Messgerät kann auch eine Schnittstelle zur Anbindung eines Computers aufweisen, die über eine vierte Datenverbindung mit dem Anwendungsprozessor verbunden ist, wobei der Anwendungsprozessor dazu ausgebildet ist, gespeicherte Logbuch-Einträge und/oder das ermittelte Vergleichsergebnis der Prüfwerte und/oder Anwendungsdaten über die Schnittstelle an den Computer zu übermitteln. Dadurch können Daten vom Messgerät an einen externen Computer übermittelt werden und auf diesem z.B. mittels einer geeigneten Software ausgewertet werden.

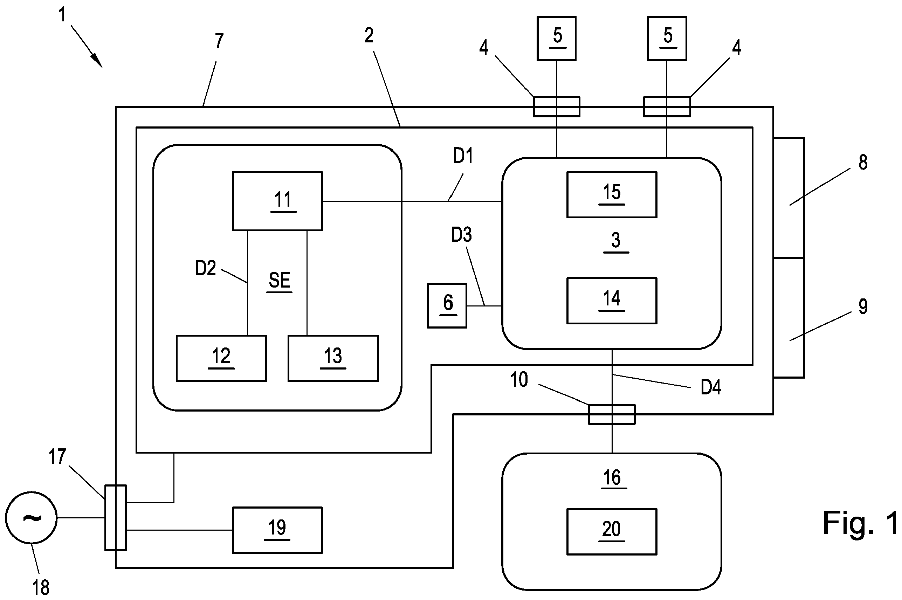

Die gegenständliche Erfindung wird nachfolgend unter Bezugnahme auf Figur 1 näher erläutert, die beispielhaft, schematisch und nicht einschränkend eine vorteilhafte Ausgestaltung eines erfindungsgemäßen Messgeräts zeigt.

In Fig. 1 ist ein beispielhaftes Messgerät 1 in einer vereinfachten schematischen Darstellung dargestellt. Das Messgerät 1 ist hier als Abgasmessgerät ausgebildet und dient zur Erfassung zumindest einer Messgröße in einem Abgasstrom, beispielsweise im Abgasstrom eines Fahrzeugs. Solche Messgeräte 1 kommen z.B. in Werkstätten bei der periodischen technischen Überprüfung von Fahrzeugen zum Einsatz, um zu überprüfen, ob die gesetzlich maximal zugelassenen Abgas-Grenzwerte eingehalten werden. Als Messgröße können dabei z.B. eine oder mehrere Abgasbestandteile vorgesehen sein, wie z.B. Kohlenmonoxid (CO), Kohlendioxid (CO₂), Kohlenwasserstoffe (HC), Partikel (PM), Stickoxide (NO, NO₂) usw. Je nach vorgesehener Verwendung kann das Messgerät 1 eine oder mehrere dieser Messgrößen erfassen. Natürlich könnte das Messgerät 1 auch dazu ausgebildet sein, noch weitere Messgrößen zu erfassen, beispielsweise Temperatur, Druck, usw. Der für die Messung erforderliche Aufbau und die Funktion der Messung sind grundsätzlich bekannt, weshalb an dieser Stelle keine detaillierte Beschreibung erfolgt. Nachfolgend werden daher nur die für die Erfindung wesentlichen Merkmale des Messgeräts 1 beschrieben.

Wie eingangs erwähnt ist die Erfindung aber nicht auf Abgasmessgeräte beschränkt. Unter dem Messgerät 1 sind im Rahmen der Erfindung natürlich auch andere Messgeräte zu verstehen, mit denen eine oder mehrere Messgrößen erfasst werden können, insbesondere Messgeräte zur Verwendung in einer KFZ-Werkstätte zur Erfassung einer oder mehrerer Messgrößen an einem Fahrzeug.

Das Messgerät 1 weist eine Steuerungseinheit 2 mit einem Anwendungsprozessor 3 zur Steuerung des Messgeräts 1 auf, insbesondere zur Steuerung der für die Erfassung der Messgrößen erforderlichen Funktionen. Der Anwendungsprozessor 3 kann beispielsweise einen bekannten Mikrocontroller oder Mikroprozessor aufweisen. Am Messgerät 1, beispielsweise an einem Gehäuse 7 des Messgeräts 1, können ein oder mehrere Sensorschnittstellen 4 vorgesehen sein, an welche jeweils ein Sensor 5 angeschlossen werden kann. Beim dargestellten Abgasmessgerät kann als Sensor beispielsweise eine Abgassonde vorgesehen sein, die in das Endrohr eines Fahrzeugs eingeführt werden kann. Bei anderen Messgeräten 1 können entsprechend der zu erfassenden Messgröße ein oder mehrere geeignete Sensoren vorgesehen sein. Es muss natürlich nicht zwingend eine Sensorschnittstelle 4 vorgesehen sein, sondern ein Sensor 5 könnte auch fest, also nicht lösbar, mit dem Messgerät 1 verbunden sein. Die vom Sensor 5 erfassten Messwerte einer Messgröße werden über geeignete Sensorleitungen an den Anwendungsprozessor 3 übermittelt und von diesem verarbeitet.

Ein Sensor 5 könnte aber prinzipiell auch im Gehäuse 7 des Messgeräts 1 angeordnet sein. In diesem Fall könnte beim dargestellten Abgasmessgerät z.B. ein Teil des Abgasstroms über eine geeignete Rohrleitung dem Sensor 5 zugeführt werden. In der Steuerungseinheit 2 ist vorzugsweise auch ein Anwendungsspeicher 6 zum Speichern von Anwendungsdaten, insbesondere von Messdaten der erfassten Messwerte vorgesehen, die über eine geeignete dritte Datenverbindung D3 mit dem Anwendungsprozessor 3 verbunden ist. Der Anwendungsspeicher 6 kann beispielsweise in Form eines geeigneten elektronischen Datenspeichers ausgebildet sein. Neben den Messdaten können natürlich noch weitere Anwendungsdaten gespeichert werden, wie nachfolgend noch näher erläutert wird.

Vorzugsweise weist das Messgerät 1 auch eine Anzeigeeinrichtung 8 auf, die in geeigneter Weise mit der Steuerungseinheit 2, insbesondere mit dem Anwendungsprozessor 3, verbunden ist. In einer einfachen Ausführungsform kann die Anzeigeeinrichtung 8 beispielsweise lediglich eine oder mehrere Signallampen aufweisen, beispielsweise Leuchtdioden unterschiedlicher Farbe. In einer vorteilhaften Ausführungsform weist die Anzeigeeinrichtung 8 jedoch einen Bildschirm auf. Das Messgerät 1 weist weiters vorzugsweise eine geeignete Benutzerschnittstelle 9 auf, über die die Funktionen des Messgeräts 1 von einem Benutzer steuerbar sind. Die Benutzerschnittstelle 9 ist in geeigneter Weise mit der Steuerungseinheit 2, insbesondere mit dem Anwendungsprozessor 3 verbunden. Die Benutzerschnittstelle 9 ist in Fig. 1 als von der Anzeigeeinrichtung 8 getrennte Einheit dargestellt. In diesem Fall könnte die Die Benutzerschnittstelle 9 beispielsweise eine Tastatur oder dergleichen aufweisen. Natürlich könnten die Anzeigeeinrichtung 8 und die Benutzerschnittstelle 9 aber auch als gemeinsame Einheit ausgeführt sein, insbesondere als Touchscreen. Der Touchscreen kann dann sowohl für die Anzeige, als auch für die Benutzereingabe verwendet werden.

Erfindungsgemäß ist in der Steuerungseinheit 2 zusätzlich zum Anwendungsprozessor 3 ein separater Sicherheitsprozessor 11 vorgesehen ist, der über eine erste Datenverbindung D1 mit dem Anwendungsprozessor 3 verbunden ist. Der Sicherheitsprozessor 11 kann wiederum einen geeigneten Mikrocontroller oder Mikroprozessor aufweisen. Weiters ist in der Steuerungseinheit 2 ein Sicherheitsspeicher 12 vorgesehen, der eine geeignete zweite Datenverbindung D2 mit dem Sicherheitsprozessor 11 verbunden ist. Der Sicherheitsspeicher 12 kann wiederum z.B. einen elektronischen Datenspeicher umfassen. Vorzugsweise ist die Speicherkapazität des Sicherheitsspeichers 12 auf die Lebensdauer des Messgeräts 1 ausgelegt und ausreichend groß gewählt, sodass über der Lebensdauer immer genügend Speicher verfügbar ist.

Der Sicherheitsprozessor 11 ist erfindungsgemäß dazu ausgebildet, einen Logbuch-Eintrag im Sicherheitsspeicher 12 zu speichern, wenn eine, die Erfassung der Messgröße betreffende, Änderung an der Steuerungseinheit 2 vorgenommen wird, wie nachfolgend noch im Detail erläutert wird. Dadurch, dass der Anwendungsprozessor 3 keinen Zugriff auf den Sicherheitsspeicher 12 hat, wird gewährleistet, dass die gespeicherten Logbuch-Einträge auch nicht nachträglich manipuliert werden können. Ein Logbuch-Eintrag kann dabei beispielsweise in Form einer geeigneten Protokolldatei, auch logfile genannt, gespeichert werden.

Vorzugsweise ist in der Steuerungseinheit 2 eine Echtzeituhr 13 vorgesehen, die in geeigneter Weise mit dem Sicherheitsprozessor 11 verbunden ist und der Sicherheitsprozessor 11 ist dazu ausgebildet, die Logbuch-Einträge mit einem durch die Echtzeituhr 13 ermittelten Zeitstempel zu versehen. Ein Zeitstempel enthält dabei vorzugsweise das aktuelle Datum und die Uhrzeit der vorgenommenen Änderung. Wie in Fig.1 angedeutet ist, können der Sicherheitsprozessor 11, der Sicherheitsspeicher 12 und ggf. die Echtzeituhr 13 beispielsweise in einer geeigneten Hardware in Form einer Sicherheitseinheit SE integriert sein, welche Teil der Steuerungseinheit 2 ist. Die Sicherheitseinheit SE ist dabei eine vom Anwendungsprozessor 3 getrennte Einheit und es sind lediglich der Sicherheitsprozessor 11 und der Anwendungsprozessor 3 über die erste Datenverbindung D1 verbunden. Die gesamte Steuerungseinheit 2 könnte beispielsweise als eine gemeinsame Hardware-Komponente ausgebildet sein oder die einzelnen Komponenten (Anwendungsprozessor 3, Sicherheitsprozessor 11, Sicherheitsspeicher 12, Anwendungsspeicher 6, Echtzeituhr 13) könnten auch als getrennte Hardware-Komponenten ausgeführt sein.

Der Anwendungsprozessor 3 enthält vorzugsweise auch eine Anwendungsfirmware 14, welche die grundlegenden Funktionen des Messgeräts 1 steuert. Eine Firmware ist bekanntermaßen eine grundlegende Betriebssoftware, die funktional fest mit der Hardware, hier dem Anwendungsprozessor 3, verbunden ist. Eine, die Erfassung der Messgröße betreffende, Änderung an der Steuerungseinheit 2, die zu einem automatischen Logbuch-Eintrag führt, kann beispielsweise eine Änderung der Anwendungsfirmware 14 umfassen. Das bedeutet, dass immer dann, wenn die Anwendungsfirmware 14 durch einen Benutzer (autorisiert oder nicht autorisiert) aktualisiert wird, wird vom Sicherheitsprozessor 11 ein zugehöriger Logbuch-Eintrag im Sicherheitsspeicher 12 gespeichert wird.

Damit ist zu jeder Zeit nachvollziehbar, ob und wann die Anwendungsfirmware 14 verändert wurde und es kann geprüft werden, ob diese Änderung Auswirkungen auf die Erfassung oder Auswertung der vom Sensor 5 erfassten Messgrößen hat. Um zu verhindern, dass ggf. unbefugte Änderungen an der Funktion des Sicherheitsprozessors 11 vorgenommen werden, ist es weiters vorteilhaft, wenn der Sicherheitsprozessor 11 eine unveränderbare Sicherheitssoftware oder Sicherheitsfirmware aufweist. Die Sicherheitssoftware oder Sicherheitsfirmware kann beispielsweise vom Hersteller programmiert und implementiert werden und ist danach nicht mehr veränderbar.

Der Anwendungsprozessor 3 enthält in vorteilhafter Weise auch einen Bootloader 15, der dazu ausgebildet ist, eine allfällige Änderung der Anwendungsfirmware 14 durchzuführen. Unter dem Bootloader ist in bekannter Weise der Code zu verstehen, der bei der Inbetriebnahme des Messgeräts 1 als erstes ausgeführt wird, also noch vor der Anwendungsfirmware 14. Um Manipulationen zu verhindern weist der Bootloader 15 vorzugsweise einen unveränderbaren Bootloader-Code auf. Der Bootloader-Code kann ebenfalls vom Hersteller programmiert und implementiert werden und ist nachträglich durch einen Benutzer nicht mehr veränderbar. Wenn von einem Benutzer eine Änderung der Anwendungsfirmware 14 durchgeführt wird, dann erzeugt der Bootloader 15 vorzugsweise einen Prüfwert für die geänderte Anwendungsfirmware 14 und übermittelt den Prüfwert an den Sicherheitsprozessor 11.

Der Sicherheitsprozessor 11 speichert den erhaltenen Prüfiniert dann im Sicherheitsspeicher 12. Der Prüfwert kann beispielsweise ein, mittels zyklischer Redundanzprüfung (cyclic redundancy check - CRC) erzeugter, CRC-Prüfiniert sein. Bei jeder Inbetriebnahme des Messgeräts 1 ermittelt der Bootloader 15 in vorteilhafter Weise jeweils vor dem Start der Anwendungsfirmware 14 einen Prüfwert für die aktuelle Anwendungsfirmware 14 des Anwendungsprozessor 3 und vergleicht diesen mit dem zuletzt im Sicherheitsspeicher 12 gespeicherten Prüfiniert, um daraus eine Vergleichsergebnis zu ermitteln, beispielsweise eine CRC-Prüfsumme.

Nachdem das Vergleichsergebnis ermittelt wurde, kann das Vergleichsergebnis beispielsweise mittels der Anzeigeeinrichtung 8 für einen Benutzer des Messgeräts 1 visualisiert werden. Dadurch kann der Benutzer verifizieren, ob ggf. eine Änderung an der Anwendungsfirmware 14 durchgeführt wurde. Die Anzeigeeinrichtung 8 könnte in einer einfachen Ausführungsform beispielsweise lediglich zwei Signallampen, z.B. LED's, unterschiedlicher Farbe (z.B. grün und rot) aufweisen. Wenn das Vergleichsergebnis ergibt, dass der aktuell ermittelte Prüfwert für die Anwendungsfirmware 14 dem zuletzt im Sicherheitsspeicher 12 gespeicherten Prüfwert entspricht, dann bedeutet das, dass zwischenzeitlich keine Änderung an der Anwendungsfirmware 14 durchgeführt wurde.

Dies kann beispielsweise mittels der grünen LED signalisiert werden. Wenn durch das Vergleichsergebnis eine Abweichung zwischen den Prüfwerten erkannt wird, dann bedeutet das, dass die Anwendungsfirmware 14 zwischenzeitlich geändert wurde. Dies kann beispielsweise mittels der roten LED signalisiert werden. Dadurch erkennt der Benutzer in einfacher Weise, ob eine Änderung erfolgt ist und kann prüfen, ob diese Änderung zulässig war oder ob es ggf. eine unbefugte Manipulation gegeben hat. Natürlich ist dies nur beispielhaft und die Anzeige könnte auch anders erfolgen. Wenn die Anzeigeeinrichtung 8 beispielsweise einen Bildschirm oder Touchscreen aufweist, dann können die Prüfwerte und/oder das Vergleichsergebnis und ggf. Anwendungsdaten in geeigneter Weise am Bildschirm oder Touchscreen angezeigt werden. Dadurch kann eine verbesserte Visualisierung ermöglicht werden und es können mehr Informationen dargestellt werden.

Zusätzlich oder alternativ zur Anzeigeeinrichtung 8 oder zur Benutzerschnittstelle 9 kann das Messgerät 1 auch eine Schnittstelle 10 zur Anbindung eines Computers 16 aufweisen, die über eine geeignete vierte Datenverbindung D4 mit der Steuerungseinheit 2, insbesondere mit dem Anwendungsprozessor 3, verbunden ist. Die Schnittstelle 10 kann wiederum am Gehäuse 7 vorgesehen sein und kann z.B. in Form einer seriellen Schnittstelle, beispielsweise RS-232, oder in Form einer USB-Schnittstelle ausgeführt sein. Der Anwendungsprozessor 3 kann dann gespeicherte Logbuch-Einträge und/oder das ermittelte Vergleichsergebnis der Prüfwerte und/oder Anwendungsdaten über die Schnittstelle 10 an einen externen Computer 16 übermitteln. Am Computer 16 können die Daten mittels einer geeigneten Software 20 verwaltet, ausgewertet und gespeichert werden. Die Software 20 kann beispielsweise ebenfalls vom Hersteller des Messgeräts 1 zur Verfügung gestellt werden und entsprechend auf das Logbuch zugreifen.

In vorteilhafter Weise wird ein Logbuch-Eintrag aber nicht nur dann erstellt und im Sicherheitsspeicher 12 gespeichert, wenn die Anwendungsfirmware 14 verändert wurde. Ein Logbuch-Eintrag wird vorzugsweise auch dann erstellt und gespeichert, wenn Änderungen der im Anwendungsspeicher 6 gespeicherten Anwendungsdaten durchgeführt werden, welche die Erfassung der Messgröße betreffen. Solche Anwendungsdaten können beispielsweise bereits gespeicherte Messdaten der erfassten Messgröße sein, und/oder Kalibrierparameter des Messgeräts und/oder Metadaten über die gespeicherten Messdaten. Wenn das Messgerät 1 dazu geeignet ist, dass gespeicherte Messdaten nachträglich von einem Benutzer noch geändert oder gelöscht werden können, könnte das unter Umständen auch missbräuchlich verwendet werden. Es ist daher vorteilhaft, wenn bei solchen Änderungen ebenfalls ein (nicht veränderbarer) Logbuch-Eintrag erstellt wird, um die Änderung später nachvollziehen zu können.

Die Änderung muss aber nicht zwangsläufig die Messdaten direkt betreffen, sondern könnte beispielsweise auch eine Änderung von Metadaten der Messdaten sein. Solche Metadaten können z.B. bestimmte Zusatzinformationen zu den gespeicherten Messdaten sein, wie z.B. der Benutzer, der die Messung durchgeführt hat, Datum und Uhrzeit der Messung, Ort der Messung, Randbedingungen der Messung, für die Messung verwendete Kalibrierparameter, Informationen zum Prüfling, an dem die Messung durchgeführt wurde, usw. Auch eine Änderung der Metadaten könnte ggf. missbräuchlich verwendet werden, sodass es vorteilhaft sein kann, wenn auch bei Änderungen an den Metadaten ein zugehöriger Logbuch-Eintrag erstellt und im Sicherheitsspeicher 12 gespeichert wird.

In gleicher Weise kann dies bei einer Änderung von Kalibrierparametern durchgeführt werden. Unter Kalibrierparametern sind dabei solche Parameter zu verstehen, mit welchen die Genauigkeit der Erfassung der Messgröße Messgeräts 1 eingestellt werden kann, um zu gewährleisten, dass die Messung tatsächlich physikalisch korrekt ist. Aufgrund von äußeren Einflussfaktoren, Alterung, usw. kann es über im Betrieb des Messgeräts 1 vorkommen, dass der Messwert der erfassten Messgröße von einem zu erwarteten Wert abweicht. Man spricht in der Messtechnik auch von Drift oder Langzeitdrift.

Behördlich zugelassene Messgeräte 1 im Allgemeinen und insbesondere auch Abgasmessgeräte werden daher in der Regel in bestimmten gesetzlich vorgegebenen zeitlichen Abständen kalibriert, um zu gewährleisten, dass die durch das Messgerät erfassten Messwerte in einem vorgegebenen Toleranzbereich um den zu erwartenden Messwert liegen. Die Kalibrierung kann über verschiedene Kalibrierparameter erfolgen, beispielsweise hinterlegte Kennwerte, Kennlinien oder Kennfelder. Daraus ist ersichtlich, dass eine Änderung an den Kalibrierparametern ggf. zu einer falschen Erfassung der Messgröße führen kann und folglich zu verfälschten Messdaten. Es ist daher vorteilhaft, wenn auch bei einer, z.B. im Zuge der Kalibrierung des Messgeräts 1 durchgeführten, Änderung der Kalibrierparameter ein Logbuch-Eintrag erzeugt und im Sicherheitsspeicher 12 gespeichert wird.

Gemäß einer weiteren vorteilhaften Ausführungsform kann der Anwendungsprozessor 3 die genannten Anwendungsdaten, z.B. die Kalibrierparameter, auch an den Sicherheitsprozessor 11 übermitteln und der Sicherheitsprozessor 11 kann die erhaltenen Anwendungsdaten im Sicherheitsspeicher 12 speichern. Im Sicherheitsspeicher 12 gespeicherte Anwendungsdaten könne wiederum an den Anwendungsprozessor 3 übermittelt werden. Dadurch können beispielsweise Kalibrierparameter sicher gespeichert und wieder abgerufen werden, beispielsweise um diese bei einer Änderung wieder auf einen vorherigen Stand zu bringen.

Allgemein ist es vorteilhaft, wenn die Logbuch-Einträge neben Datum und Uhrzeit auch Informationen über die Art der durchgeführten Änderung an der Steuerungseinheit 2 und/oder einen Benutzernamen des Benutzers, der die Änderung an der Steuerungseinheit durchgeführt hat, und/oder einen Status der Steuerungseinheit 2 vor der Änderung an der Steuerungseinheit enthält. Unter der Art der durchgeführten Änderung ist beispielsweise zu verstehen, in welchem Bereich der Steuerungseinheit 2 eine Änderung erfolgt ist, z.B. ob die Änderung an der Anwendungsfirmware 14 oder an den Anwendungsdaten, beispielsweise den Messdaten, Kalibrierparametern oder den Metadaten, durchgeführt wurde. Der Benutzername kann beispielsweise dann gespeichert werden, wenn das Messgerät 1 für die Verwendung eine Anmeldung erfordert, z.B. mit Benutzername und Passwort. Unter dem Status der Steuerungseinheit 2 vor der Änderung ist beispielsweise eine eindeutige Kennung der Anwendungsfirmware 14, z.B. in Form einer Versionsnummer, zu verstehen oder eine eindeutige Kennung der verwendeten Kalibrierparameter, z.B. in Form eines Verweises auf eine Kennlinie oder ein Kennfeld.

Am Messgerät 1, beispielsweise am Gehäuse 7, ist vorzugsweise auch ein Versorgungsanschluss 17 zur Verbindung des Messgeräts 1 mit einer Energiequelle 18, beispielsweise einer herkömmlichen Haushaltssteckdose, vorgesehen. Dadurch kann das Messgerät 1, insbesondere die Steuerungseinheit 2, mit der für den Betrieb erforderlichen Energie versorgt werden. Zusätzlich kann im Messgerät 1, insbesondere im Gehäuse 7, auch ein elektrischer Energiespeicher 19, beispielsweise eine wiederaufladbare Batterie, vorgesehen sein. Der Energiespeicher 19 kann dabei einerseits mit dem Versorgungsanschluss 17 verbunden sein, um den Energiespeicher 19 zu laden und kann andererseits mit der Steuerungseinheit 2 verbunden sein. Dadurch kann das Messgerät 1 beispielsweise zumindest temporär ohne externe Energieversorgung betrieben werden.

## Patentansprüche

1. Messgerät (1) zur Erfassung zumindest einer Messgröße, wobei das Messgerät (1) eine Steuerungseinheit (2) mit einem Anwendungsprozessor (3) zur Steuerung des Messgeräts (1) aufweist, **dadurch gekennzeichnet, dass** in der Steuerungseinheit (2) ein zusätzlicher Sicherheitsprozessor (11) vorgesehen ist, der über eine erste Datenverbindung (D1) mit dem Anwendungsprozessor (3) verbunden ist und über eine zweite Datenverbindung (D2) mit einem Sicherheitsspeicher (12) verbunden ist **und dass** der Sicherheitsprozessor (11) dazu ausgebildet ist, einen Logbuch-Eintrag im Sicherheitsspeicher (12) zu speichern, wenn eine, die Erfassung der Messgröße betreffende, Änderung an der Steuerungseinheit (2) vorgenommen wird.

2. Messgerät (1) nach Anspruch 1, **dadurch gekennzeichnet, dass** in der Steuerungseinheit (2) eine Echtzeituhr (13) vorgesehen ist und dass der Sicherheitsprozessor (11) dazu ausgebildet ist, den Logbuch-Eintrag mit einem durch die Echtzeituhr (13) ermittelten Zeitstempel zu versehen, wobei der Zeitstempel vorzugsweise Datum und Uhrzeit enthält.

3. Messgerät (1) nach Anspruch 1 oder 2, **dadurch gekennzeichnet, dass** der Anwendungsprozessor (3) eine Anwendungsfirmware (14) enthält und dass die, die Erfassung der Messgröße betreffende, Änderung an der Steuerungseinheit (2) eine Änderung der Anwendungsfirmware (14) umfasst, wobei vorzugsweise der Anwendungsprozessor (3) einen Bootloader (15) enthält, der dazu ausgebildet ist, die Änderung der Anwendungsfirmware (14) durchzuführen und dass der Bootloader (15) einen unveränderbaren Bootloader-Code aufweist.

4. Messgerät (1) nach Anspruch 3, **dadurch gekennzeichnet, dass** der Bootloader (15) dazu ausgebildet ist, bei einer Änderung der Anwendungsfirmware (14) einen Prüfwert für die geänderte Anwendungsfirmware (14) zu erzeugen und an den Sicherheitsprozessor (11) zu übermitteln und dass der Sicherheitsprozessor (11) dazu ausgebildet ist, den erhaltenen Prüfiniert im Sicherheitsspeicher (12) zu speichern, wobei vorzugsweise der Bootloader (15) dazu ausgebildet ist, bei der Inbetriebnahme des Messgeräts (1) vor dem Start der Anwendungsfirmware (14) einen Prüfwert für die aktuelle Anwendungsfirmware (14) zu ermitteln und mit dem zuletzt im Sicherheitsspeicher (12) gespeicherten Prüfwert zu vergleichen, um ein Vergleichsergebnis zu ermitteln.

5. Messgerät (1) nach einem der Ansprüche 1 bis 4, **dadurch gekennzeichnet, dass** der Sicherheitsprozessor (11) eine unveränderbare Sicherheitssoftware oder unveränderbare Sicherheitsfirmware aufweist.

6. Messgerät (1) nach einem der Ansprüche 1 bis 5, **dadurch gekennzeichnet, dass** in der Steuerungseinheit (2) ein Anwendungsspeicher (6) zum Speichern von Anwendungsdaten vorgesehen ist, der über eine dritte Datenverbindung (D3) mit dem Anwendungsprozessor (3) verbunden ist und dass die, die Erfassung der Messgröße betreffende, Änderung an der Steuerungseinheit (2) eine Änderung der im Anwendungsspeicher (6) gespeicherten Anwendungsdaten umfasst, wobei vorzugsweise die Anwendungsdaten Messdaten der erfassten Messgröße und/oder Kalibrierparameter des Messgeräts (1) und/oder Metadaten über die erfassten Messdaten enthalten.

7. Messgerät (1) nach Anspruch 6, **dadurch gekennzeichnet, dass** der Anwendungsprozessor (3) dazu ausgebildet ist, Anwendungsdaten an den Sicherheitsprozessor (11) zu übermitteln und dass der Sicherheitsprozessor (11) dazu ausgebildet ist, die erhaltenen Anwendungsdaten im Sicherheitsspeicher (12) zu speichern und/oder im Sicherheitsspeicher (12) gespeicherte Anwendungsdaten an den Anwendungsprozessor (3) zu übermitteln.

8. Messgerät (1) nach einem der Ansprüche 1 bis 7, **dadurch gekennzeichnet, dass** der Logbuch-Eintrag die Art der durchgeführten Änderung an der Steuerungseinheit (2) und/oder einen Benutzernamen des Benutzers, der die Änderung an der Steuerungseinheit (2) durchgeführt hat, und/oder einen Status der Steuerungseinheit (2) vor der Änderung an der Steuerungseinheit (2) enthält.

9. Messgerät (1) nach einem der Ansprüche 1 bis 8, **dadurch gekennzeichnet, dass** das Messgerät (1) eine Anzeigeeinrichtung (8) aufweist, die dazu ausgebildet ist, gespeicherte Logbuch-Einträge und/oder das ermittelte Vergleichsergebnis der Prüfwerte und/oder Anwendungsdaten anzuzeigen, wobei die Anzeigeeinrichtung vorzugsweise zumindest eine Signallampe oder einen Bildschirm aufweist.

10. Messgerät (1) nach einem der Ansprüche 1 bis 9, **dadurch gekennzeichnet, dass** das Messgerät (1) eine Benutzerschnittstelle (9) aufweist, über die Funktionen des Messgeräts (1) von einem Benutzer steuerbar sind, wobei die Benutzerschnittstelle (9) vorzugsweise einen Touchscreen oder eine Tastatur aufweist.

11. Messgerät (1) nach einem der Ansprüche 1 bis 10, **dadurch gekennzeichnet, dass** das Messgerät (1) eine Schnittstelle (10) zur Anbindung eines Computers (16) aufweist, die über eine vierte Datenverbindung (D4) mit dem Anwendungsprozessor (3) verbunden ist und dass der Anwendungsprozessor (3) dazu ausgebildet ist, gespeicherte Logbuch-Einträge und/oder das ermittelte Vergleichsergebnis der Prüfwerte und/oder Anwendungsdaten über die Schnittstelle (10) an den Computer (16) zu übermitteln.

12. Verfahren zum Betreiben eines, zur Erfassung zumindest einer Messgröße ausgebildeten, Messgerätes (1), wobei das Messgerät (1) durch eine, einen Anwendungsprozessor (3) aufweisende, Steuerungseinheit (2) gesteuert wird, **dadurch gekennzeichnet, dass** mit einem in der Steuerungseinheit (2) zusätzlich vorgesehenen und mit dem Anwendungsprozessor (3) kommunizierenden Sicherheitsprozessor (11) ein Logbuch-Eintrag in einem ausschließlich mit dem Sicherheitsprozessor kommunizierenden Sicherheitsspeicher (12) gespeichert wird, wenn eine, die Erfassung der Messgröße betreffende, Änderung an der Steuerungseinheit (2) durchgeführt wird, wobei der Logbuch-Eintrag vorzugsweise einen durch eine Echtzeituhr (13) ermittelten Zeitstempel und/oder die Art der durchgeführten Änderung und/oder einen Benutzernamen des Benutzers, der die Änderung durchgeführt hat, und/oder einen Status der Steuerungseinheit (2) vor der Änderung enthält.

13. Verfahren nach Anspruch 12, **dadurch gekennzeichnet, dass** ein Logbuch-Eintrag gespeichert wird, wenn eine Anwendungsfirmware (14) des Anwendungsprozessors (3) geändert wird oder wenn in einem Anwendungsspeicher (6) der Steuerungseinheit (2) gespeicherte Anwendungsdaten geändert werden, die vorzugsweise Messdaten der erfassten Messgröße und/oder Kalibrierparameter des Messgeräts (1) und/oder Metadaten über die erfassten Messdaten enthalten.

14. Verfahren nach Anspruch 12 oder 13, **dadurch gekennzeichnet, dass** die Änderung der Anwendungsfirmware (14) von einem im Anwendungsprozessor (3) implementierten Bootloader (15) mit einem unveränderbaren Bootloader-Code durchgeführt wird, wobei bei einer Änderung der Anwendungsfirmware (14) der Bootloader (15) vorzugsweise einen Prüfwert für die geänderte Anwendungsfirmware (14) erzeugt und an den Sicherheitsprozessor (11) übermittelt und der Sicherheitsprozessor (11) den erhaltenen Prüfiniert im Sicherheitsspeicher (12) speichert, wobei besonders vorzugsweise der Bootloader (15) bei der Inbetriebnahme des Messgeräts (1) vor dem Start der Anwendungsfirmware (14) einen Prüfwert für die aktuelle Anwendungsfirmware (14) ermittelt und mit dem zuletzt im Sicherheitsspeicher (12) gespeicherten Prüfwert vergleicht und ein Vergleichsergebnis ermittelt.

15. Verfahren nach einem der Ansprüche 12 bis 14, **dadurch gekennzeichnet, dass** das gespeicherte Logbuch-Einträge und/oder das ermittelte Vergleichsergebnis der Prüfwerte und/oder Anwendungsdaten auf einer Anzeigeeinrichtung angezeigt werden.
